# EUROPÄISCHE PATENTSCHRIFT

(11) **EP 2 111 161 B1**
(45) Veröffentlichungstag und Bekanntmachung des Hinweises auf die Patenterteilung: **04.04.2012**
(21) Anmeldenummer: 08708030.5
(22) Anmeldetag: 21.01.2008
(51) Int. Cl.: A61B 6/00

(54) **VORRICHTUNG UND VERFAHREN FÜR EINE MEDIZINISCHE DIAGNOSE**
APPARATUS AND METHOD FOR A MEDICAL DIAGNOSIS
DISPOSITIF ET PROCÉDÉ POUR UN DIAGNOSTIC MÉDICAL

(30) Priorität: 23.01.2007 DE 102007003380
(43) Veröffentlichungstag der Anmeldung: 28.10.2009
(73) Patentinhaber: Siemens Aktiengesellschaft, 80333 München (DE)
(72) Erfinder: HÖRNIG, Mathias, 91052 Erlangen (DE)
(86) Internationale Anmeldenummer: PCT/EP2008/050626
(87) Internationale Veröffentlichungsnummer: WO 2008/090118

(56) Entgegenhaltungen:
- EP-A- 1 428 473
- WO-A-94/06352
- WO-A-98/49939
- WO-A-2006/061357
- US-B1- 6 292 531

## Beschreibung

Die Erfindung, wie in den Ansprüchen 1 und 5 definiert ist, bezieht sich auf eine Vorrichtung und ein dazugehöriges Verfahren für eine medizinische Diagnose mit einer Hochenergiestrahlquelle. Von dieser Hochenergiestrahlquelle werden hochenergetische Photonen emittiert und von einem der Hochenergiestrahlquelle zugeordneten Hochenergiedetektor detektiert. Zwischen Hochenergiestrahlquelle und Hochenergiedetektor wird ein zu untersuchendes Objekt angeordnet.

Eine derartige Vorrichtung und/oder ein Verfahren ist auf dem Gebiet der Mammographie allgemein bekannt. Dabei handelt es sich um eine Vorrichtung und ein Verfahren zur Untersuchung der sogenannten Mamma. Die Mammographie wird insbesondere zur Früherkennung von Brustkrebs verwendet. Dazu wird die zu untersuchende Mamma zwischen einer Kompressionsplatte und einer Detektorplatte komprimiert und mit Hilfe von Röntgenstrahlen, die von einer Röntgenquelle erzeugt werden, durchleuchtet. Eine Fixierung und/oder eine Ausrichtung bzw. Anordnung des zu untersuchenden Objektes auf der Detektorplatte bzw. Detektoreinheit erfolgt durch eine Verschiebung der Kompressionsplatte.

An die Verschiebung der Kompressionsplatte gekoppelt erfolgt eine Umlenkung des Röntgenstrahls durch eine automatische Nachführung der an der Röntgenstrahlaustrittsöffnung der Röntgenquelle angeordneten Blenden. Die Umlenkung des Röntgenstrahls bringt jedoch den Nachteil mit sich, dass bei Gewebeteilen die in Randbereichen der Mamma liegen aufgrund der Ablenkung des Röntgenzentralstrahls eine mindere Auflösung vorliegt. Die verminderte Auflösung führt zu einer reduzierten Bildqualität.

Aus der internationalen Anmeldung WO 94/06352 ist eine Mammographieanlage zur Brustaufnahme und ein dazugehöriger Biopsieapparat offenbart. Bei dieser Mammographieanlage wird in seiner Gesamtheit das Mammographiegerät bestehend aus Detektor, Kompressionsplatte und Röntgeneinheit verschoben.

Der Erfindung liegt die Aufgabe zugrunde, einen Weg zu zeigen, wie ein Diagnosesystem der eingangs genannten Art mit einer optimalen Bildqualität geschaffen werden kann.

Die Aufgabe wird durch die im Patentanspruch 1 oder 6 angegebenen Merkmale gelöst.

Vorteilhafte Ausgestaltungen der Erfindung sind in den Unteransprüchen angegeben.

Die erfindungsgemäße Vorrichtung und das dazugehörige Verfahren ist derart ausgebildet, dass eine Strahlungsquelle in Bezug auf Randbereiche einer Detektoreinheit derart ausrichtbar ist, dass die hochenergetischen Strahlen der Strahlungsquelle, bezogen auf den Zentralstrahl der Strahlungsquelle, auch symmetrisch zu diesem verteilt auf der Detektoreinheit auftreffen.

Die Erfindung bringt den Vorteil mit sich, dass auf individuelle Gegebenheiten und Erfordernisse bei der Untersuchung der Mamma eingegangen werden kann.

Die Erfindung bringt den Vorteil mit sich, dass ein optimaler Einsatz der von der Hochleistungsquelle emittierten Strahlung erzielbar ist.

Die Erfindung bringt den Vorteil mit sich, dass neben wählbaren Kompressionsplatten mit bestimmten Geräteangulationen, in Verbindung mit einer Abstimmung zwischen der zentralen, symmetrischen Ausrichtung von Röntgenstrahl zum Detektor, ein optimaler Einsatz der von der Hochleistungsquelle emittierten Strahlung erzielbar ist.

Die Erfindung bringt den Vorteil mit sich, dass alle Bereiche des zu betrachtenden Objekts für eine Diagnose in einem Bild darstellbar sind.

Die Erfindung bringt den Vorteil mit sich, dass eine gleich bleibend optimierte Bildqualität auch in Randbereichen gewährleistet ist.

Die Erfindung bringt den weiteren Vorteil mit sich, dass durch Sensoren eine exakte Positionierung der Strahlungsquelle durchführbar ist.

Die Erfindung bringt den weiteren Vorteil mit sich, dass die Diagnoseeinheit durch eine Reset-Taste automatisch in eine zentrierte Default-Position gefahren werden kann.

Die Erfindung bringt den Vorteil mit sich, dass durch ein Aktivieren eines Lichtfeldes auf dem Objekt die Strahlungsquelle ausrichtbar ist.

Die Erfindung bringt den Vorteil einer optimierten Gain-Korrektur mit sich.

Die Erfindung bringt neben dem Vorteil einer leichten Bedienung durch eine Slidertaste den weiteren Vorteil mit sich, dass ein Workflow durch diese Funktion verbessert wird.

Die Erfindung bringt den Vorteil mit sich, dass Bildbearbeitungsoperationen zur Bereinigung von Auflösungsverlusten, Bildunschärfe oder Intensitätsabfall am Rand eines zu untersuchenden Objektes entfallen.

Die Erfindung soll im Folgenden mittels in Zeichnungen dargestellten Ausführungsbeispielen näher erläutert werden.

Dabei zeigen:
- Figur 1: eine Draufsicht auf eine schematische Darstellung,
- Figur 2: eine Vorderansicht,
- Figur 3: eine Draufsicht auf eine weitere Darstellung,
- Figur 4: eine Vorderansicht der weiteren Darstellung,
- Figur 5: eine Vorderansicht eines Mammographiegerätes in einemBetriebszustand und
- Figur 6: eine Vorderansicht des Mammographiegerätes in einem weiteren Betriebszustand.

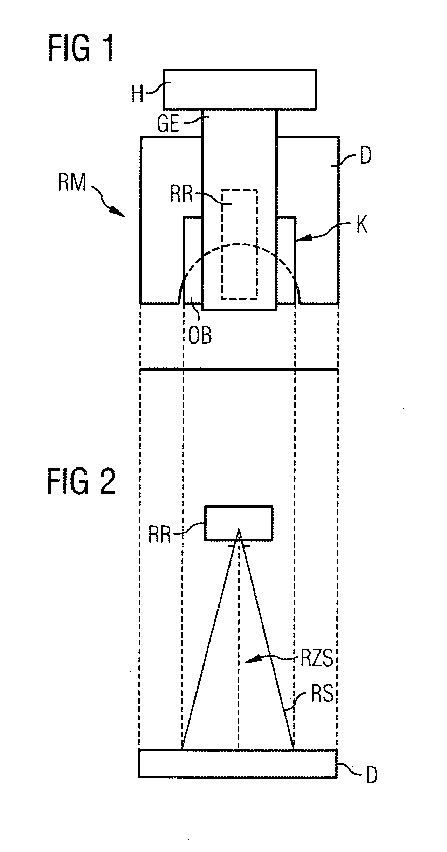

In Figur 1 ist eine Draufsicht einer schematischen Darstellung eines Mammographiegerätes RM wiedergegeben.

Die an einer Halterung H angeordnete Gehäuseeinheit GE weist eine Röntgenstrahlung emittierende Röntgenstrahlungsquelle RR auf. Ferner umfasst das Mammographiegerät RM eine Flachdetektoren aufweisende Detektoreinheit bzw. Detektionsplatte D sowie eine Kompressionsplatte K. Eine Mamma OB, im nochfolgenden auch Objekt genannt, ist zwischen Detektionsplatte D und Kompressionsplatte K ausricht- bzw. fixierbar.

In Figur 2 ist die Vorderansicht der in Figur 1 wiedergegebenen schematischen Darstellung des Mammographiegerätes RM abgebildet. Gezeigt wird der Strahlungsbereich der von der Röntgenröhre RR abgegebenen Röntgenstrahlen RS sowie der Verlauf des Röntgen-Zentralstrahls RZS. Angedeutet ist ebenso der Bereich, in dem der Röntgenstrahl RS auf die Detektorplatte D auftrifft.

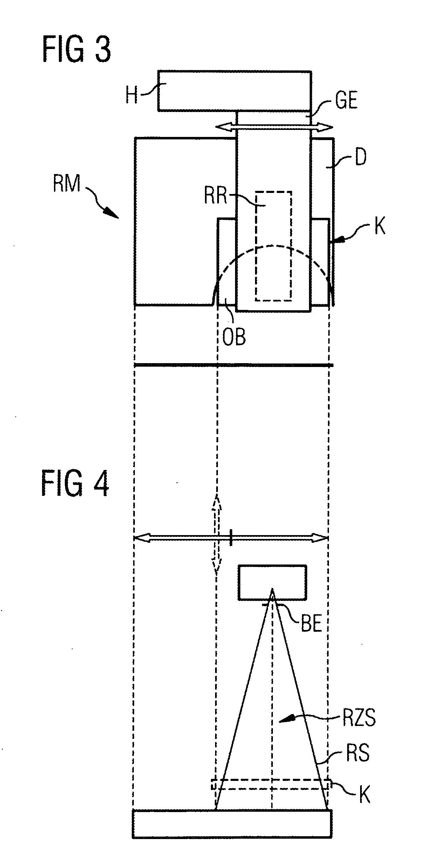

In Figur 3 ist die Ausprägung eines Mammographiegerätes RM gemäß der Erfindung wiedergegeben. Hier wird die Röntgenröhre RR und die Kompressionsplatte K als eine Einheit betrachtet. Ein Collimator zur Filterung der Röntgenstrahlung RS kann dieser Einheit zugeordnet sein. Eine die Röntgenröhre RR aufnehmende Gehäuseeinheit GE ist mit der Halterung H fixierbar verbunden. Diese Gehäuseeinheit GE ist horizontal verschiebbar. Eine vertikale Veränderung der Gehäuseeinheit GE kann ebenso vorgenommen werden.

In Figur 4 ist eine Vorderansicht des Mammographiegerätes RM aus Figur 3 wiedergegeben. Die unter Figur 3 angesprochene Einheit kann entsprechend der angedeuteten Bewegungsrichtungen derart verschoben werden, dass für den Zeitraum der Untersuchung der Patient eine Position einnehmen kann, in der jeder zu untersuchende Bereich der Mamma mit einem ungebeugten Röntgenstrahlbündel erreichbar ist.

Die Ausgestaltung ermöglicht durch die Verwendung von verschieden großen Kompressionsplatten bei bestimmten Geräteangulationen einen verbesserten Patientenzugang. Die Geräteangulation kann beispielsweise mit einem bei Diagnosegeräten üblichen C-Bogen durchgeführt werden. Die örtliche Ausrichtung der die Röntgenröhre aufnehmende Gehäuseeinheit GE erfolgt beispielsweise mittels elektrischer Antriebsmittel. Die Positionierung der Einheit kann entweder über eine Slider-taste stufenlos oder mittels einer Shifttaste zu Positionierungspunkten verschoben werden. Eine Fixierung der Gehäuseeinheit GE, in Verbindung mit der Kompressionseinheit, erfolgt durch Loslassen der Slider-Taste. Eine Fixierung kann auch rasterförmig erfolgen, wobei die Postition auch akustisch angezeigt werden kann. Mit dem Verschieben der Einheit E kann gleichzeitig ein automatisches Aktivieren eines Lichtfeldes am Collimator erfolgen, so dass unmittelbar eine visuelle Darstellung des angewählten Untersuchungsbereichs bzw. Bildbereichs zur Verfügung steht.

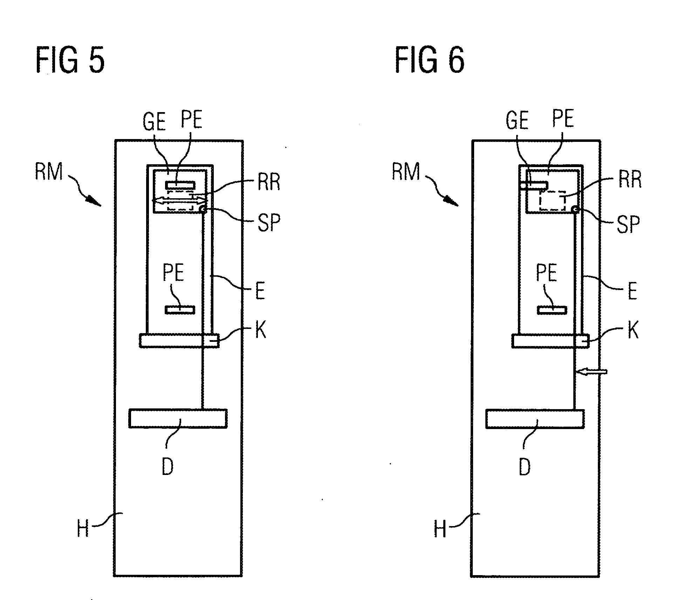

In den Figuren 5 und 6 ist schematisch eine Ausgestaltung eines Mammographiegerätes RM mit der bereits angesprochenen Einheit E in Vorderansicht dargestellt. Diese Einheit E besteht unter Anderem aus der Gehäuseeinheit GE, der in der Gehäuseeinheit integrierten Röntgenstrahlungsquelle RR und der Kompressionsplatte K. Die die Auslenkung der Einheit E begrenzenden Positionselemente PE sind beispielsweise eingezeichnet. Figur 5 zeigt das Mammographiegerät RM mit der Einheit E in einer Ausgangs- oder Ruheposition. In Figur 6 ist die Einheit E nach rechts verschoben. Eine Positionierung der Einheit E kann durch einen im Gehäuse GE angeordneten Stepper SP erfolgen. Dieser kann exakt die Positionierung der Röntgenröhre RR bzw. der von dieser ausgehenden Röntgenstrahlung zum Detektor D bestimmen. Eine Feinsteuerung der Einheit E in oder aus einer Arbeitsposition kann dabei unterstützt per Lasertechnik oder per Reflexionsmessung erfolgen. Eine Ausrichtung kann ebenso mittels Memoryfunktion erfolgen. Neben der elektrischen und elektronischen Positionierung ist auch eine mechanisch durchführbare Positionierung möglich.

Eine Kalibrierung kann zumindest in der zentralen, linkssowie rechtszentralen Positionen durchgeführt werden. Für mögliche Zwischenpositionen ist zudem eine lineare Interpolation mittels einem Gain-Mappen möglich.

In einer alternativen Ausführung erfolgt die Slider-Funktion stufenlos, lediglich begrenzt durch eine links- und rechtsseitige Endposition. Die links- und rechtsseitige Endposition kann durch den Randbereich des Röntgenstrahls an der Detektorkante vorgegeben sein. Die Positionserkennung kann mittels Lasertechnik erfolgen. Durch eine Reset-Funktion kann eine zentrale Mittelposition der Röntgenröhre wieder angefahren werden. Die Position der Einheit E kann vom Betriebssystem der Diagnoseeinheit gespeichert und für erneute Untersuchungen abgerufen werden.

In einer weiteren Ausgestaltung kann die Information aus einer Positionierung dafür genutzt werden, um einen vörselektierten Detektorbereich zu fokussieren und Zeit für den Daten-Read-Out, Daten-Transfer und das Daten-Processing einzusparen.

In einer weiteren Ausgestaltung werden Röhre und Blendeneinheit synchron verschoben. Das Betriebssystem der Diagnoseeinheit RM verfügt über eine automatische Positionserkennung sowie eine Reset-Funktion mit der Röhre und Blendeneinheit BE inklusiv Kompressionsplatte K, womit jeweils in die zentrale Ausgangsposition gefahren werden kann. Das Fokussieren mittels des Steppers SP kann entweder per Laserdiode oder per Reflektionsmessung oder mechanisch mittels einer MemoryFunktion erfolgen.

### Bezugszeichenliste

- Halterung: H
- Gehäuseeinheit: GE
- Röntgenstrahlungsquelle: RR
- Einheit: E
- Detektoreinheit/Detektorplatte: D
- Objekt/Mamma: OB
- Kompressionsplatte: K
- Mammographiegerät: RM
- Röntgenstrahl: RS
- Röntgen-Zentralstrahl: RZS
- Positionselemente: PE
- Stepper: SP

## Patentansprüche

1. Vorrichtung zur Mammographie mit einer Strahlungsquelle (RR) und einer Detektoreinheit (D), wobei die von einer Strahlungsquelle (RR) abgegebene hochenergetische Strahlung (RS) nach einem zu durchleuchtenden Objekt (OB) auf die Detektoreinheit (D) trifft,
**dadurch gekennzeichnet,**
**dass** die Strahlungsquelle (RR) derart positionierbar ist, dass auch bei einer Ausrichtung auf ein zu durchleuchtendes Objekt (OB) im Randbereich der Detektoreinheit (D) die hochenergetischen Strahlen, bezogen auf den Zentralstrahl (RZS) der Strahlungsquelle (RR), symmetrisch zu diesem verteilt auf der Detektoreinheit (D) auftreffen,
**dass** die Strahlungsquelle (RR) und eine Kompressionsplatte (K) eine Einheit (E) bilden, wobei sich durch ein Verändern der Position der Strahlungsquelle (RR) die Position der Kompressionsplatte (K), getrennt von der Detektoreinheit (D), ebenfalls verändert.

2. Vorrichtung nach Anspruch 1,
**dadurch gekennzeichnet,**
**dass** die Position der Strahlungsquelle (RR) horizontal und/oder vertikal veränderbar und diese in der jeweiligen Position fixierbar ist.

3. Vorrichtung nach Anspruch 1,
**dadurch gekennzeichnet,**
**dass** die Positionierung der Einheit (E) durch die Randbereiche der Detektoreinheit (D) vorgebbar ist.

4. Vorrichtung nach Anspruch 1,
**dadurch gekennzeichnet,**
**dass** eine Positionierung der Einheit (E) mittels Lasersteuerung durchführbar ist.

5. Verfahren zur Mammographie, wobei hochenergetische Strahlung (RS) nach einem zu durchleuchtenden Objekt (OB) auf eine Detektoreinheit (D) trifft,
**dadurch gekennzeichnet,**
**dass** in Randbereichen der Detektoreinheit (D) die hochenergetischen Strahlen, bezogen auf einen Zentralstrahl, auch symmetrisch zu diesem verteilt auf der Detektorplatte (D) auftreffen,
**dass** durch ein Verändern der Position der die hochenergetischen Strahlen abgebenden Strahlungsquelle (RR) die Position der Kompressionsplatte (K), getrennt von der Detektoreinheit(D), ebenfalls verändert wird.

6. Verfahren nach Anspruch 5,
**dadurch gekennzeichnet,**
**dass** die Position der Strahlungsquelle (RR) horizontal und/oder vertikal veränderbar und diese in der jeweiligen Position fixiert werden.

7. Verfahren nach Anspruch 6,
**dadurch gekennzeichnet,**
**dass** eine Positionierung mittels Lasersteuerung durchgeführt wird.

## Claims

1. Mammography apparatus having a radiation source (RR) and a detector unit (D), wherein the high-energy radiation emitted by a radiation source (RR) strikes the detector unit (D) downstream of an object (OB) to be fluoroscopically examined,
**characterised in that**
the radiation source (RR) can be positioned in such a manner that,
in the case of alignment with an object (OB) to be fluoroscopically examined in the border region of the detector unit (D) the high-energy beams also strike said detector unit (D) with symmetrical distribution relative to the central beam (ZRS) of the radiation source (RR),
the radiation source (RR) and a compression plate (K) form a unit (E), wherein, by changing the position of the radiation source (RR), the position of the compression plate (K) likewise changes, independently of the detector unit (D).

2. Apparatus according to claim 1,
**characterised in that**
the position of the radiation source (RR) can be changed horizontally and/or vertically and it can be fixed in the respective position.

3. Apparatus according to claim 1,
**characterised in that**
the positioning of the unit (E) can be predetermined by the border regions of the detector unit (D).

4. Apparatus according to claim 1,
**characterised in that**
positioning of the unit (E) can be performed by means of laser control.

5. Mammography method, wherein high-energy radiation (RS) strikes a detector unit (D) downstream of an object (OB) to be fluoroscopically examined,
**characterised in that**
in border regions of the detector unit (D) the high-energy beams also strike the detector plate (D) with symmetrical distribution relative to a central beam
by changing the position of the radiation source (RR) emitting the high-energy beams, the position of the compression plate (K) is likewise changed, independently of the detector unit (D).

6. Method according to claim 5,
**characterised in that**
the position of the radiation source (RR) can be changed horizontally and/or vertically and it is fixed in the respective position.

7. Method according to claim 6,
**characterised in that**
positioning can be performed by means of laser control.

## Revendications

1. Dispositif de mammographie comprenant une source de rayonnement (RR) et une unité de détection (D), le rayonnement hautement énergétique (RS) émis par une source de rayonnement (RR) arrivant sur l'unité de détection (D) après un objet (OB) à radiographier, **caractérisé**
**en ce que** la source de rayonnement (RR) peut être positionnée de telle sorte que, même lorsqu'elle est alignée avec un objet (OB) à radiographier, les rayons hautement énergétiques arrivent sur l'unité de détection (D) en étant répartis de manière symétrique par rapport au rayon central (RZS) de la source de rayonnement (RR) dans la zone de bord de l'unité de détection (D),
**en ce que** la source de rayonnement (RR) et une plaque de compression (K) forment une unité (E), une modification de la position de la source de rayonnement (RR) modifiant également la position de la plaque de compression (K), séparément de l'unité de détection (D).

2. Dispositif selon la revendication 1, **caractérisé en ce que** la position de la source de rayonnement (RR) peut être modifiée horizontalement et/ou verticalement et celle-ci peut être fixée dans la position respective.

3. Dispositif selon la revendication 1, **caractérisé en ce que** le positionnement de l'unité (E) peut être prédéterminé par les zones de bord de l'unité de détection (D).

4. Dispositif selon la revendication 1, **caractérisé en ce qu'**un positionnement de l'unité (E) peut être exécuté au moyen d'une commande laser.

5. Procédé de mammographie, un rayonnement hautement énergétique (RS) arrivant sur une unité de détection (D) après un objet (OB) à radiographier, **caractérisé en ce que** les rayons hautement énergétiques arrivent sur la plaque de détection (D) en étant eux aussi répartis de manière symétrique par rapport à un rayon central dans les zones de bord de l'unité de détection (D),
**en ce qu'**une modification de la position de la source de rayonnement (RR) qui émet les rayons hautement énergétiques modifie également la position de la plaque de compression (K), séparément de l'unité de détection (D).

6. Procédé selon la revendication 5, **caractérisé en ce que** la position de la source de rayonnement (RR) peut être modifiée horizontalement et/ou verticalement et celle-ci est fixée dans la position respective.

7. Procédé selon la revendication 6, **caractérisé en ce qu'**un positionnement est exécuté au moyen d'une commande laser.
